# EUROPÄISCHE PATENTSCHRIFT

(11) **EP 1 955 668 B1**
(45) Veröffentlichungstag und Bekanntmachung des Hinweises auf die Patenterteilung: **04.04.2012**
(21) Anmeldenummer: 07101887.3
(22) Anmeldetag: 07.02.2007
(51) Int. Cl.: A61B 19/00

(54) **Verfahren und Vorrichtung zum Ermitteln von Ausrichtungsinformationen während der sonographischen navigierten Reposition von Knochenfragmenten**
Method and device for the determination of alignment information during sonographically navigable repositioning of bone fragments
Procédé et dispositif pour la détermination des informations d'alignement pendant le repositionnement navigué sonographiquement de fragments d'os

(43) Veröffentlichungstag der Anmeldung: 13.08.2008
(73) Patentinhaber: BrainLAB AG, 85622 Feldkirchen (DE)
(72) Erfinder: Maier, Georg, 81541 München (DE); Bertram, Michael, 85570 Markt Schwaben (DE)
(74) Vertreter: Schwabe - Sandmair - Marx

(56) Entgegenhaltungen:
- EP-A2- 0 950 379
- WO-A-01/47413
- WO-A-02/00093
- WO-A-02/062249
- US-A1- 2006 241 388
- HOFSTETTER R ET AL: "COMPUTER-ASSISTED FLUOROSCOPY-BASED REDUCTION OF FEMORAL FRACTURES AND ANTETORSION CORRECTION" COMPUTER AIDED SURGERY, TAYLOR & FRANCIS INC., PHILADELPHIA, PA, US, Bd. 5, 2000, Seiten 311-325, XP001001432 ISSN: 1092-9088

## Beschreibung

Die vorliegende Erfindung betrifft ein Verfahren und eine Vorrichtung zur sonographischen navigierten Einrichtung von dislozierten Frakturen, wobei hierzu charakteristische Landmarken auf Fragmenten des Knochens erfasst und deren Positionen ermittelt werden und basierend auf den ermittelten Positionen der Landmarken die Fragmente relativ zueinander ausgerichtet und in der ausgerichteten Position miteinander verbunden werden können.

Bei der operativen Behandlung einer Fraktur ist es von großer Bedeutung, die durch die Fraktur entstandenen Fragmente wieder genau relativ zueinander auszurichten und zu positionieren, um die relativ zueinander ausgerichteten Fragmente wieder so miteinander verbinden zu können, dass ein den natürlichen anatomischen Gegebenheiten entsprechendes Zusammenwachsen der Fragmente ermöglicht wird. Werden beispielsweise bei einer Fraktur des Femurs die durch den Bruch entstandenen Fragmente des Femurs nicht richtig relativ zueinander positioniert und verdreht relativ zueinander fixiert, kann dies auf Grund der starken Veränderung der natürlichen anatomischen Struktur und Biomechanik zu Komplikationen, wie Haltungsfehlern oder Bewegungseinschränkungen sowie Überlastungen von Gelenkstrukturen des Patienten führen. Die anatomisch verdrehte Position der proximalen und distalen gelenkbildenden Strukturen relativ zueinander entlang der Schaftachse wird beim Femur durch den so genannten Antetorsionswinkel beschrieben, der bei Frakturen des Femur wiederhergestellt werden muss. Andere Röhrenknochen können nach Frakturen ebenso eine rotatorische Abweichung von ihrer anatomischen Längsachse haben, die bei der Frakturversorgung korrigiert werden muss.

Bei klinisch bekannten Verfahren werden in dem weit von der Fraktur entfernten artikularen Gebiet Landmarken durch verschiedene meist bildgebende Verfahren aufgenommen und gegeneinander ausgerichtet. Bei einem bekannten Verfahren werden für die Bestimmung des Femurantetorsionswinkels Bilder des Femurkopfes, des Trochanter major und der Femurkondylen benötigt. Zur Ausrichtung der Fragmente werden in den bekannten Verfahren entweder anatomische Mittelwerte verwendet oder es werden die anatomischen Gegebenheiten der gesunden Seite untersucht und auf die Seite der Fraktur übertragen. Probleme treten zum einen dann auf, wenn beide Seiten durch Frakturen betroffen sind und demnach keine gesunde Seite als Vorlage zur Verfügung steht. Zum anderen können auch dadurch Probleme auftreten, dass die anatomischen Gegebenheiten einer Seite nicht direkt auf die andere Seite übertragbar sind, da die meisten Menschen keinen exakt symmetrischen Knochenbau besitzen und die Werte beispielsweise für die Femurantetorsion in einem Kollektiv stark variieren. Folglich kann es bei bekannten Verfahren zu Ungenauigkeiten und Fehlstellungen kommen, selbst wenn eine gesunde Seite als Vorlage zur Verfügung steht, da sich die anatomischen Gegebenheiten beider Seiten nur in seltenen Fällen exakt gleichen.

Die WO 2005/115246 A1 betrifft ein Verfahren und eine Vorrichtung zur eingriffslosen Bestimmung markanter Strukturen des menschlichen oder tierischen Körpers, bei dem man den Körper im Bereich der markanten Struktur mittels eines Ultraschall aussendenden und Ultraschall empfangenden Ultraschallkopfes mit Ultraschallstrahlung bestrahlt, der an der markanten Struktur reflektierte Ultraschallstrahlung empfängt und eine der Laufzeit der reflektieren Strahlung entsprechende Abbildung auf einer Anzeige darstellt, und um eine Positionsbestimmung der markanten Struktur im Körper vornehmen zu können, wird vorgeschlagen, dass man die Lage und Orientierung des Ultraschallkopfes und des Körpers im Bereich der markanten Struktur mit Hilfe von am Ultraschallkopf und am Körper festgelegten Markierelementen mittels eines Navigationssystems bestimmt, dass man auf der Anzeige eine markante Struktur auswählt, aufgrund der Strahlrichtung des Ultraschallkopfes und der gemessenen Laufzeit der Ultraschallstrahlung die Lage und Orientierung der markanten Struktur relativ zum Ultraschallkopf und aus der Lage und Orientierung des Ultraschallkopfes relativ zum Körper die Lage und Orientierung der markanten Struktur relativ zum Körper bestimmt.

Die US 6,379,302 betrifft eine Überlagerung von Navigationsinformationen auf Ultraschallbilder, wobei ein chirurgisches Instrumentennavigationssystem eine Ultraschallmaschine und einen mit der Ultraschallmaschine verbundenen Computer umfasst. Ein Speicher ist mit dem Computer verbunden und enthält Computeranweisungen, welche, wenn sie von dem Computer ausgeführt werden, den Computer dazu veranlassen, ein Icon zu erzeugen, welches das chirurgische Instrument mit einer Spitze und die Trajektorie des chirurgischen Instruments repräsentiert, und das Icon einem Echtzeit-Ultraschallbild zu überlagern, welches eine solche Bildebene hat, dass bei Schneiden der Bildebene durch das chirurgische Instrument das Format der Trajektorie des chirurgischen Instruments geändert wird, um das Schneiden der Ultraschallbildebene durch das chirurgische Instrument darzustellen. Das System umfasst auch einen mit der Ultraschallmaschine verbundenen Lokalisierer, und eine mit dem Computer verbundene Anzeige zur Anzeige des dem Echtzeitbild überlagerten generierten Icons.

Aus der US 2006/0241388 A1 ist ein Verfahren und ein rechnerbasiertes System zur Unterstützung der Reparatur einer Knochenfraktur bekannt. Der frakturierte Knochen umfasst ein erstes und ein zweites Knochensegment, wobei die Knochensegmente zu einem dritten Knochenelement symmetrisch sind. Das Verfahren umfasst folgende Schritte: Bestimmen einer ersten Darstellung der Oberfläche des ersten Knochensegments, einer zweiten Darstellung, in einer Anfangsposition, des zweiten Knochensegments, und einer dritten Darstellung des dritten Knochenelements; Bestimmen einer vierten Darstellung des ersten und des zweiten Knochensegments in einem intakten oder gesunden Knochen, wobei die vierte Darstellung mittels Matching an die erste Darstellung angepasst ist; Anpassen der zweiten Darstellung an die vierte Darstellung in einer Endposition mittels Matching; Bereitstellen der Information betreffend die Differenz zwischen der Anfangs- und der Endposition mittels eines Verbindungselements.

Aus dem Aufsatz "Computer-Assisted Fluoroscopy-Based Reduction of Femoral Fractures and Antetorsion Correction" von R. Hofstetter et al. ist ein chirurgisches Navigationssystem basierend auf fluoroskopischen Bildern oder Röntgenbildern zum Ermitteln von Informationen für eine Fixierung einer femoralen Fraktur bekannt. An jedem der zwei Knochenfragmente des frakturierten Knochens, sowie an dem Bildverstärker des Röntgengeräts und an dem chirurgischen Werkzeug, wie zum Beispiel Bohrer oder Nagel, werden Marker angeordnet. Zum Ausrichten der Knochenfragmente zueinander wird eine Torsions-Korrektur zwischen den Knochenfragmenten ausgeführt, wobei der Antetorsionswinkel berechnet wird. Ein Antetorsionswinkel an dem frakturierten Knochen wird mit einem Antetorsionswinkel eines intakten Knochens verglichen. Basierend auf diesem Vergleich wird die Torsions-Korrektur der Knochenfragmente durchgeführt.

Es ist eine Aufgabe der vorliegenden Erfindung, die Nachteile aus dem Stand der Technik zu überwinden. Es ist weiter eine Aufgabe der vorliegenden Erfindung, ein Verfahren zum Ermitteln von Ausrichtungsinformationen und eine Vorrichtung zum Ausrichten zweier korrespondierender Fragmente einer Fraktur relativ zueinander bereitzustellen, welche eine einfache, schnelle und genaue Positionsangabe der Fragmente während der Ausrichtung der Fragmente relativ zueinander und damit eine genaue Fixierung der Fragmente relativ zueinander ermöglichen.

Diese Aufgabe wird durch den Gegenstand der unabhängigen Ansprüche 1, 5 und 7 gelöst. Vorteilhafte Ausführungsformen ergeben sich aus den abhängigen Ansprüchen.

Es werden Ausrichtungsinformationen ermittelt, welche angeben, wie durch Fraktur entstandene Fragmente eines Knochens, beispielsweise eines Femurs, einer Tibia, eines Humerus, einer Scapula, einer Clavicula, eines Calcaneus, eines Os Metatarsale, eines Os Metacarpale oder eines Os Naviculare, relativ zueinander ausgerichtet oder positioniert werden sollen, so dass die Fragmente so fixiert werden können, dass diese wieder richtig relativ zueinander positioniert zusammenwachsen können, um die Anatomie und korrekte Biomechanik des Knochens wiederherzustellen.

Hierzu wird eine Ultraschallvorrichtung so bezüglich des Körperteils, an welchem sich die Fraktur befindet, positioniert, dass mittels der Ultraschallvorrichtung Bereiche in einem Gebiet um die Fraktur erfasst werden können, so dass charakteristische Landmarken des Knochens, welche sich auf dem Umfang oder der Außenseite des Knochens befinden, von der Ultraschallvorrichtung erfasst werden können. Vorzugsweise wird vor dem Erfassen der Landmarken festgelegt, welche Landmarken des Knochens von der Ultraschallvorrichtung erfasst werden sollen, wobei sich die Landmarken in einem Gebiet um die Fraktur des Knochens befinden. Beispielsweise können sich die Landmarken wenige Millimeter bis zu einige Zentimeter entfernt von der Fraktur befinden, insbesondere jedoch nicht an den von der Fraktur abgewandten Enden des jeweiligen Knochens. Es werden also nicht die zur Bestimmung anatomischer Messwerte definierten Landmarken vermessen, sondern lokale Landmarken mit Bezug zur Fraktur sonografisch dargestellt und deren charakteristische Ausrichtung einander angepasst.

Zur Aufnahme der lokalen Landmarken wird die Ultraschallvorrichtung so bezüglich des Knochens positioniert, dass die Ultraschallvorrichtung mindestens eine charakteristische Landmarke eines ersten Fragments erfasst und die damit korrespondierende charakteristische Landmarke eines zweiten Fragments erfasst. Auf den erfassten Aufnahmen des Knochens können die charakteristischen Landmarken auf dem ersten und dem zweiten Fragment identifiziert werden.

Dabei ist an der Ultraschallvorrichtung eine vorzugsweise Infrarot-Strahlung emittierende oder reflektierende Trackingreferenz, wie ein Referenzstern, angeordnet, mit Hilfe dessen ein Navigationssystem die Ultraschallvorrichtung verfolgen kann, so dass die Position und/oder Orientierung der Ultraschallvorrichtung im Raum, beispielsweise von einer in das Navigationssystem integrierten oder mit dem Navigationssystem verbundenen Recheneinheit, ermittelt werden kann. Damit können die Position und/oder Orientierung der Landmarken bezüglich der Ultraschallvorrichtung z.B. von der Recheneinheit ermittelt werden. Auch an dem ersten Teil des Knochens und an dem zweiten Teil des Knochens ist jeweils eine Trackingreferenz, wie ein Referenzstern, angeordnet. Mit dem Navigationssystem kann somit auch die Position und/oder Orientierung des ersten und des zweiten Teils des Röhrenknochens im Raum ermittelt werden, so dass auch die Position und/oder Orientierung der erfassten charakteristischen Landmarken bezüglich der an den Fragmenten angeordneten Referenzsterne und damit relativ zu dem Knochen ermittelt werden können. Die Lage und Orientierung des ersten und zweiten Knochenteils, der Ultraschallvorrichtung und der Landmarken im Raum kann somit ermittelt oder berechnet werden.

Basierend auf der ermittelten Position und/oder Orientierung der mindestens einen Landmarke auf dem ersten Fragment und der ermittelten Position und/oder Orientierung der mindestens einen Landmarke auf dem zweiten Fragment können Ausrichtungsinformationen ermittelt und ausgegeben werden, welche angeben, wie das erste und das zweite Fragment relativ zueinander ausgerichtet werden sollen. Beispielsweise können absolute Positionsinformationen ermittelt werden, welche beispielsweise mittels Koordinaten oder mittels absoluter Positionswerte bezüglich eines definierten Koordinatensystems im Raum angeben, wie das erste und das zweite Fragment positioniert werden sollen, um eine korrekte Ausrichtung der Fragmente bezüglich einander zu erreichen. Auch können relative Platzierungsinformationen angegeben werden, wie Differenz- oder Unterschiedswerte in mehreren Freiheitsgraden zwischen den Fragmenten, welche beispielsweise angeben, wie die Position eines der Fragmente gewählt werden soll, um eine korrekte Ausrichtung relativ zu dem anderen Fragment zu erreichen. Weiter können auch Ausrichtungsveränderungsinformationen ermittelt und ausgegeben werden, welche angeben, wie die Position des ersten und die Position des zweiten Fragments verändert werden müssen, um eine korrekte Ausrichtung der Fragmente relativ zueinander zu erreichen.

Bevorzugt erfolgt die Ausrichtung des ersten und des zweiten Fragments beispielsweise eines Röhrenknochens relativ zueinander durch Drehung des ersten und des zweiten Teils zumindest nahezu um die Längsachse des Röhrenknochens. Auch kann ein Marknagel in den Markkanal eingebracht oder eingeschoben werden, um welchen die Fragmente zumindest nahezu um die Längsachse des Knochens gedreht werden können. Auch können zusätzlich translatorische Bewegungen der Fragmente und weitere rotatorische Bewegungen um andere Achsen durchgeführt werden, um die Fragmente relativ zueinander zu positionieren.

Vorzugsweise können der erste und der zweite Teil des Röhrenknochens so relativ zueinander ausgerichtet werden, dass die mindestens eine charakteristische Landmarke auf dem ersten Fragment und die mindestens eine charakteristische Landmarke auf dem zweiten Fragment des Röhrenknochens in einem vorbestimmten oder ermittelbaren Lageverhältnis relativ zueinander liegen. Beispielsweise kann von einem Benutzer eingegeben werden, in welchem Lageverhältnis die charakteristischen Landmarken liegen sollen. Zum Beispiel kann ein Benutzer eingeben, wie die Landmarken relativ zueinander positioniert sein sollen, dass sie sich z.B. gegenüber liegen sollen, oder ein Benutzer kann die Richtung und den Betrag eingeben, welche den Abstand oder das Lageverhältnis der Landmarken relativ zueinander beschreiben. Basierend auf dem eingegebenen oder vorgegebenen Lageverhältnis der Landmarken relativ zueinander, können der erste und der zweite Knochenteil relativ zueinander ausgerichtet werden. Auch können z.B. von der Recheneinheit sinnvolle Lageverhältnisse der Landmarken relativ zueinander ermittelt oder vorgeschlagen werden, aus denen ein Benutzer beispielsweise das gewünschte Lageverhältnis auswählen kann, so dass basierend auf diesem Lageverhältnis oder den mehreren vorgeschlagenen Lageverhältnissen die Fragmente relativ zueinander positioniert werden können.

Beispielsweise wird als charakteristische Landmarke des ersten und des zweiten Fragments eines Röhrenknochens die linea aspera des Femurs verwendet. Die linea aspera ist eine longitudinal verlaufende Knochenleiste des dorsalen Oberschenkelknochens. Sie besteht aus einer lateralen und einer medialen Lippe (Labium). Die linea aspera wird bevorzugt dann als Landmarke verwendet, wenn die Fraktur im Bereich liegt, in dem sie besonders gut zu erkennen ist (insbesondere im mittleren Drittel des Femurs). Beispielsweise kann die linea aspera von der Recheneinheit auch automatisch als mögliche Landmarke vorgeschlagen werden, wenn der Recheneinheit bekannt ist oder in die Recheneinheit eingegeben wurde, dass die Fraktur im mittleren Bereich, insbesondere im mittleren Drittel des Femurs liegt. Vorzugsweise kann die Ultraschallvorrichtung bei einer im mittleren Bereich des Femurs liegenden Fraktur manuell oder automatisch in den mittleren Bereich des Femurs in die Nähe der Fraktur bewegt werden, um die linea aspera des ersten Teils und des zweiten Teils des Röhrenknochens als charakteristische Landmarken aufzunehmen. Die linea aspera kann in den Ultraschallaufnahmen manuell von einem Benutzer, semi-automatisch oder mittels Bilderkennungsalgorithmen automatisch identifiziert oder erkannt werden, so dass unter Berücksichtigung der ermittelten Position und/oder Orientierung der Ultraschallvorrichtung und der Fragmente die Position und/oder Orientierung der linea aspera ermittelt werden kann. Unter Berücksichtigung der Position und/oder Orientierung der linea aspera kann z.B, von der Recheneinheit ermittelt und ausgegeben werden, wie die linea aspera des ersten Fragments und die linea aspera des zweiten Fragments relativ zueinander bewegt und ausgerichtet werden sollen, um eine korrekte Ausrichtung der Fragmente relativ zueinander zu gewährleisten. Hierfür können beispielsweise Ausrichtungsinformationen ausgegeben werden, welche angeben, wie die Fragmente relativ zueinander bewegt oder gedreht werden müssen, damit die linea aspera des ersten Fragments direkt in die linea aspera des zweiten Fragments übergeht, so dass die linea aspera des aus dem ersten und dem zweiten Knochenfragment gebildeten Röhrenlmochens eine durchgehende Gerade ohne Knickstellen oder Unstetigkeitsstellen bildet. Durch diese Ausrichtung kann z.B. gewährleistet werden, dass der Torsionswinkel zwischen dem ersten und dem zweiten Teil des Röhrenknochens gering oder zumindest nahezu null wird und der Röhrenknochen wieder in seiner ursprünglichen anatomischen Stellung zusammenwachsen kann.

Auch können andere charakteristische Knochenstrukturen wie Linien, Wölbungen, Vertiefungen oder Kanten an Stelle der linea aspera als charakteristische Landmarke verwendet werden. Am Femur kann das die querovale Querschnittsform der distalen Metaphyse oder der exzentrische Querschnitt des Trochantermassivs sein. Es bietet sich beispielsweise auch die Tibiavorderkante, der Sulcus radialis, die distalen Humerusepicondylen oder je nach Struktur einer Fraktur auch die Frakturlinie im Knochen selbst als Landmarke im Sinne der Erfindung an.

Auch können Ultraschallvorrichtungsplatzicrungsinformationen ermittelt oder z.B. von einem Benutzer angegeben werden, welche beschreiben, an welche Position und/oder mit welcher Orientierung die Ultraschallvorrichtung mittels des Navigationssystems navigiert oder bewegt werden soll, um die mindestens eine Landmarke des ersten Fragments beispielsweise von einer ersten Position aus und die mindestens eine Landmarke des zweiten Fragments beispielsweise von einer zweiten Position aus erfassen zu können. Beispielsweise kann eine Position ermittelt oder angegeben werden, welche in Längsrichtung oder entlang der Längsachse des Röhrenknochens in einem bestimmten Gebiet um die Fraktur, z.B. in einem Abstand oder in einem Kreis mit einem Durchmesser von weniger als einem Zentimeter oder von einen, zwei, drei, vier, fünf oder mehr Zentimetern liegt, und es kann eine Orientierung ermittelt oder angegeben, so dass die Ultraschallvorrichtung z.B. senkrecht zu dem Röhrenknochen oder dem den Röhrenknochen enthaltenden Körperteil steht. Die Fraktur bestimmt durch ihre Lage und Form die Verwendbarkeit von Landmarken im Frakturbereich. Abstände können zum Beispiel angegeben werden, wenn sich die Fraktur im Bereich anatomisch genau definierter Landmarken befindet. Als lokale Landmarken definiert der Operateur vorzugsweise intraoperativ markante Knochenstrukturen. Je näher die Landmarke bei der Fraktur liegt, desto weniger Verzerrung ergibt sich, Liegt die Fraktur nicht an Landmarken, werden die Abstände zum Beispiel nur ungefähr angegeben. Basierend auf den Ultraschallvorrichtungsplatzierungsinformationen kann die Ultraschallvorrichtung manuell oder automatisch mittels des Navigationssystems an die ermittelte oder eingegebene Position mit der ermittelten oder eingegebenen Orientierung bewegt werden.

Selbstverständlich können auch mehr als zwei Fragmente eines Röhrenknochens mit dem erfindungsgemäßen Verfahren relativ zueinander ausgerichtet werden, indem jeweils zwei miteinander verbindbare Fragmente der mehr als zwei Fragmente relativ zueinander ausgerichtet und positioniert werden.

Sind die Fragmente relativ zueinander ausgerichtet, können die Fragmente an ihrer Verbindungsstelle über einen in den Markkanal eingebrachten Marknagel miteinander verbunden werden. Der Marknagel kann z.B. in den Femur von der Hüftseite in den oberen Teil des Femurs oder von der Knieseite in den unteren Teil des Femurs eingebracht weiden. Der Marknagel wird vorzugsweise so positioniert, dass der Marknagel in dem Markkanal des ersten und des zweiten Teils des Röhrenknochens liegt, so dass die Fragmente über den Marknagel miteinander verbunden werden können. Vorzugsweise kann die Position des Marknagels mittels Röntgenaufnahmen z.B. durch einen C-Bogen ermittelt werden. Basierend auf den Röntgenaufnahmen kann die Recheneinheit ermitteln, wie der Marknagel in dem Markkanal positioniert ist und wie seine Position verändert werden soll oder wie der Marknagel in dem Markkanal navigiert werden soll, um eine möglichst gute Verbindung der Fragmente zu ermöglichen. Bevorzugt enthält der Marknagel quer oder senkrecht zu der Längsachse oder dem Markkanal des Röhrenknochens verlaufende Festlegelemente, wie Löcher oder Bohrungen, über welche der Marknagel, z.B. durch in die Festlegelemente einbringbare Einführelemente, wie Stäbe oder Schrauben, festgelegt werden kann, so dass der Marknagel relativ zu den Fragmenten des Röhrenknochens fixiert werden kann. Mittels der Fixierung können auch die Fragmente des Röhrenknochens relativ zueinander fixiert oder festgesetzt werden. Dadurch kann ein Zusammenwachsen der Fragmente ermöglicht oder vereinfacht oder beschleunigt werden.

Die vorliegende Erfindung betrifft weiter ein Computerprogramm und ein Computerprogrammprodukt zur Ausführung des erfindungsgemäßen Verfahrens.

Die erfindungsgemäße Vorrichtung umfasst eine Ultraschallvorrichtung und ein Navigationssystem mit einer Recheneinheit, wie einem Computer oder einem Prozessor. Die Recheneinheit kann in das Navigationssystem integriert sein oder kann mit dem Navigationssystem drahtlos oder drahtgebunden verbunden sein. An der Ultraschallvorrichtung ist eine Trackingreferenz, wie ein Infrarot-Strahlung reflektierender oder emittierender Referenzstern, angeordnet, welche das Navigationssystem erfassen kann, so dass das Navigationssystem die Position und/oder Orientierung der Trackingreferenz und damit auch der Ultraschallvorrichtung im Raum ermitteln kann. Mit der Ultraschallvorrichtung können mittels Ultraschallstrahlung Ultraschallbilder eines Körperteils und eines in dem Körperteil liegenden Röhrenknochens aufgenommen werden. An jedem der durch Fraktur des Röhrenknochens entstandenen Fragmente wird oder ist eine Trackingreferenz, wie ein Referenzstern, angeordnet, welche das Navigationssystem erfassen kann, so dass das Navigationssystem die Position und/oder Orientierung des Referenzsterns und dadurch der Fragmente im Raum ermitteln kann.

Die Ultraschallvorrichtung wird beispielsweise manuell, semi-automatisch oder automatisch an eine Position bewegt, von der aus sie einen Bereich des ersten Fragments aufnehmen kann, in welchem sich mindestens eine charakteristische Landmarke auf dem Umfang oder auf der Außenseite des ersten Fragments befindet. Diese Landmarke kann z.B. die linea aspera des Femurs oder eine andere charakteristische Landmarke sein. Am Femur kann das beispielsweise die distale Metaphyse oder der Querschnitt des Trochantermassivs sein. Es bietet sich beispielsweise auch die Tibiavorderkante, der Sulcus radialis, die distalen Humerusepicondylen oder je nach Struktur einer Fraktur auch die Frakturlinie im Knochen selbst als Landmarke im Sinne der Erfindung an. Der Bereich, welcher von der Ultraschallvorrichtung aufgenommen wird, befindet sich bevorzugt in der Nähe der Fraktur, wie z.B. nur wenige Millimeter oder Zentimeter von der Fraktur entfernt. Nach der Aufnahme des Bereichs des ersten Fragments kann die Ultraschallvorrichtung manuell, semi-automatisch oder automatisch an eine weitere Position bewegt werden, von der aus sie einen Bereich des zweiten Fragments des Röhrenknochens aufnehmen kann, in welchem sich mindestens eine gleichartige oder andersartige charakteristische Landmarke auf dem Umfang oder der Außenseite des zweiten Fragments befindet. Die zweite Landmarke kann zu der ersten Landmarke gleichartig sein, es kann also auf beiden Fragmenten beispielsweise die linea aspera aufgenommen werden. Es können jedoch auch verschieden- oder andersartige charakteristische Landmarken von der Ultraschallvorrichtung aufgenommen werden. Die Recheneinheit kann basierend auf der ermittelten Position und/oder Orientierung der Ultraschallvorrichtung und der ermittelten Position und/oder Orientierung der Fragmente des Knochens die Position und/oder die Orientierung der Landmarken im Raum ermitteln. Dabei kann von der Recheneinheit insbesondere ermittelt werden, wie die Landmarken bezüglich des Knochens gelegen und orientiert sind. Damit ist der Recheneinheit die Position und/oder Orientierung der Landmarke auf dem Umfang des ersten Fragments relativ zu dem ersten Fragment und die Position und/oder Orientierung der Landmarke auf dem Umfang des zweiten Fragments relativ zu dem zweiten Fragment bekannt. Die Recheneinheit kann weiter basierend auf der ermittelten Position und/oder Orientierung der mindestens einen Landmarke auf der Außenseite des ersten Fragments und der ermittelten Position und/oder Orientierung der mindestens einen Landmarke auf der Außenseite des zweiten Fragments Ausrichtungsinformationen ermitteln, welche angeben, wie die Fragmente relativ zueinander ausgerichtet werden sollen, um eine korrekte Platzierung der Fragmente relativ zueinander oder einen möglichst kleinen Rotationsfehler oder eine möglichst geringe Achsabweichung der Fragmente zu erreichen.

Bevorzugt umfasst die erfindungsgemäße Vorrichtung weiter eine mit der Ultraschallvorrichtung verbundene Anzeigevorrichtung. Auf der Anzeigevorrichtung können die aufgenommenen Ultraschallbilder dargestellt werden. Weiter können auf der Anzeigevorrichtung auch die ermittelten Ausrichtungsinformationen angezeigt werden. Beispielsweise können die Ausrichtungsinformationen auch den Ultraschallbildern überlagert werden und z.B. anzeigen, wie die Position der Fragmente verändert werden soll. Beispielsweise können die Ausrichtungsinformationen als Zahlenwerte oder als Koordinaten auf der Anzeigevorrichtung angezeigt werden. Auch können die Ausrichtungsinformationen grafisch, z.B. als Pfeile dargestellt werden, welche anzeigen, wie die Fragmente relativ zueinander bewegt werden sollen.

Vorzugsweise ist das Navigationssystem so ausgebildet, dass die Ultraschallvorrichtung an bestimmte Positionen zur Aufnahme der mindestens einen Landmarke des ersten und zweiten Fragments navigiert werden kann. Die Positionen können beispielsweise manuell von einem Benutzer, wie über die Angabe von Koordinaten oder Positionswerten, angegeben werden. Auch können von der Recheneinheit Ultraschallvorrichtungsplatzierungsinformationen ermittelt werden, welche angeben, an welche Positionen und mit welcher Orientierung die Ultraschallvorrichtung zur Aufnahme der Landmarken bewegt werden soll.

Die Erfindung wird weiter anhand bevorzugter Ausführungsformen beschrieben werden. Es zeigt:

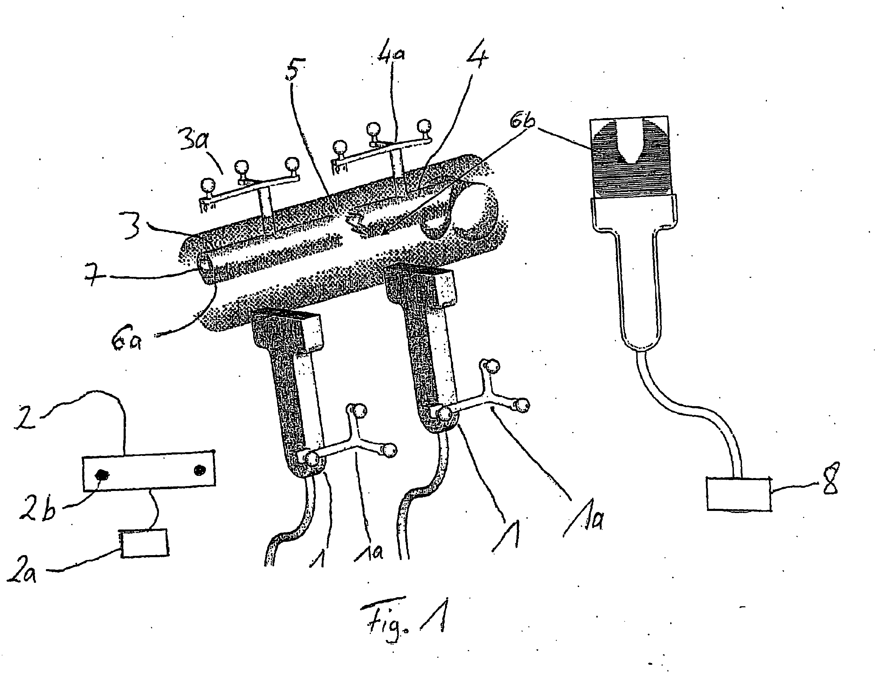

- Figur 1: eine erste Ausführungsform der vorliegenden Erfindung mit einer an zwei Positionen positionierten Ultraschallsonde zur Aufnahme der linea aspera des Femurs als Landmarke,

Figur 1 zeigt eine erste Ausführungsform der vorliegenden Erfindung mit einer Ultraschallsonde 1, einem Navigationssystem 2 mit Infrarot-Kameras 2b und einem mit dem Navigationssystem 2 verbundenen Computer 2a. An der Ultraschallsonde 1 ist ein Referenzstern 1a angeordnet, welcher von dem Navigationssystem 2 detektiert werden kann, so dass der Computer 2a die Position und Orientierung des mit der Ultraschallsonde 1 verbundenen Referenzsterns 1a und damit auch die Position und Orientierung der Ultraschallsonde 1 ermitteln kann. Weiter zeigt Figur 1 ein Femur, welches durch eine Fraktur in zwei Fragmente 3, 4 auseinander gebrochen ist. An jedem der zwei Fragmente 3, 4 ist jeweils ein Referenzstern 3a, 4a angeordnet. Das Navigationssystem 2 kann die an den Fragmenten 3, 4 angeordneten Referenzsterne 3a, 4a erfassen, so dass der Computer 2a die Position und Orientierung des mit dem ersten Knochenteil 3 verbundenen Referenzsterns 3a und die Position und Orientierung des mit dem zweiten Knochenteil 4 verbundenen Referenzsterns 4a und damit auch die Position und Orientierung des ersten 3 und zweiten Knochenteils 4 ermitteln kann.

Die Ultraschallsonde 1 wird zunächst zumindest nahezu senkrecht zu dem Femur in einer ersten Position positioniert, in welcher die Ultraschallsonde 1 den ersten Knochenteil 3 und die auf dem Umfang oder der Außenseite des ersten Knochenteils 3 angeordnete linea aspera 6a als Landmarke aufnehmen kann. Nach der Aufnahme wird die Ultraschallsonde 1 zumindest nahezu senkrecht zu dem Femur in einer zweiten Position positioniert, in welcher die Ultraschallsonde 1 den zweiten Knochenteil 4 und die auf dem Umfang oder der Außenseite des zweiten Knochenteils 4 angeordnete linea aspera 6b als Landmarke aufnehmen kann, Die linea aspera 6a, 6b des ersten 3 und des zweiten Knochenteils 4 kann in den Ultraschallaufnahmen identifiziert werden, so dass unter Berücksichtigung der ermittelten Position und Orientierung der Fragmente 3, 4 und der Ultraschallvorrichtung 1 von der Recheneinheit 2a auch die Position und Orientierung der linea aspera 6a des ersten Knochenteils 3 und der linea aspera 6b des zweiten Knochenteils 4 ermittelt werden kann. Ist der Recheneinheit 2a, z.B. durch manuelle Eingabe, bekannt, wie die linea aspera 6a, 6b der beiden Fragmente .3, 4 relativ zueinander positioniert werden soll, können von der Recheneinheit 2a aus der Position und Orientierung der Ultraschallsonde 1 und der Fragmente 3, 4 und den Angaben der gewünschten Position der linea aspera 6a, 6b der Fragmente 3, 4 relativ zueinander, Ausrichtungsinformationen ermittelt werden, welche angeben, wie die Position und Orientierung der beiden Fragmente 3, 4 relativ zueinander verändert werden sollen. Beispielsweise kann ein Benutzer angeben, dass die linea aspera 6a des ersten Knochenteils 3 direkt in die linea aspera 6b des zweiten Knochenteils 4 übergehen soll. Unter Berücksichtigung dieser Angabe und unter Berücksichtung der ermittelten Position und Orientierung der Fragmente 3, 4 und der Ultraschallvorrichtung 1 können beispielsweise Winkelwerte von der Recheneinheit 2a ermittelt und auf einem Bildschirm 8 angezeigt werden, so dass die Fragmente 3, 4 um die angezeigten Winkel werte verdreht werden können, um die Fragmente 3, 4 relativ zueinander zu positionzeren.

Sind die beiden Fragmente 3, 4 relativ zueinander ausgerichtet, wird ein Marknagel in den Markkanal 7 des Femurs so eingeschoben, dass der Marknagel beide Fragmente durch den Markkanal 7 des ersten 3 und des zweiten Fragments 4 durchdringt. Der Marknagel weist Querbohrungen oder Querlöcher auf, die Befestigungselemente aufnehmen. Durch Einbringen dieser Befestigungselemente durch das Femur in die Querlöcher des Marknagels werden die Fragmente 3, 4 relativ zu dem Marknagel und damit auch relativ zueinander fixiert, so dass sie korrekt relativ zueinander ausgerichtet zusammenwachsen können. Dadurch wird der Antetorsionswinkel im Vergleich zu herkömmlichen Verfahren so fixiert, dass die Fragmente 3, 4 zu einem Femur zusammenwachsen, welcher im Vergleich zu einem mittels herkömmlicher Verfahren widerhergestellten Femur eine größere Ähnlichkeit oder Übereinstimmung mit der ursprünglichen, natürlichen anatomischen Form des Femurs hat.

## Patentansprüche

1. Verfahren zum Ermitteln von Ausrichtungsinformationen, welche angeben, wie ein erstes Fragment (3) und ein zweites Fragment (4) eines Knochens relativ zueinander ausgerichtet werden sollen, wobei das erste (3) und das zweite Fragment (4) des Knochens an einer Verbindungsstelle (5) miteinander verbunden werden können,
a) wobei die Position und/oder Orientierung des ersten (3) und des zweiten Fragments (4) des Knochens im Raum mit Hilfe von an dem ersten Fragment (3) und an dem zweiten Fragment (4) des Knochens angeordneten Trackingreferenzen (3a, 4a) mittels eines Navigationssystems (2) ermittelt wird,
b) wobei mittels einer Ultraschallvorrichtung (1), deren Position und/oder Orientierung im Raum mit Hilfe eines an der Ultraschallvorrichtung (1) angeordneten Trackingreferenz (1a) mittels des Navigationssystems (2) ermittelt wird, mindestens eine charakteristische Landmarke (6a) auf dem Umfang oder der Außenseite des ersten Fragments (3) des Knochens in einem Bereich um die Verbindungsstelle (5) zwischen dem ersten (3) und dem zweiten Fragment (4) des Knochens, das heißt in einem Abstand von weniger als einem Zentimeter oder von ein, zwei, drei, vier, oder fünf Zentimeter zu einer Fraktur, erfasst wird und
c) wobei mittels der Ultraschallvorrichtung (1) mindestens eine korrespondierende charakteristische Landmarke (6b) auf dem Umfang oder der Außenseite des zweiten Fragments (4) des Knochens in dem Bereich um die Verbindungsstelle zwischen dem ersten (3) und dem zweiten Fragment (4) des Knochens erfasst wird,
d) wobei die Position und/oder Orientierung der Landmarken (6a, 6b) im Raum basierend auf der Position und/oder Orientierung der Ultraschallvorrichtung (1) und der Position und/oder Orientierung des ersten (3) und zweiten Fragments (4) des Knochens durch eine Rechnereinheit (2a) des Navigationssystems (2) ermittelt wird, und
e) wobei ein Lageverhältnis der Landmarken (6a, 6b) relativ zueinander durch einen Benutzer in die Rechnereinheit (2a) des Navigationssystems (2) eingegeben wird,
f) so dass, basierend auf dem vorgegeben Lageverhältnis der Landmarken (6a, 6b) und der Position und/oder Orientierung der mindestens einen Landmarke (6a) auf dem ersten Fragment (3) des Knochens und der Position und/oder Orientierung der mindestens einen Landmarke (6b) auf dem zweiten Fragment (4) des Knochens, die Ausrichtungsinformationen ermittelt werden, welche angeben, wie das erste (3) und das zweite Fragment (4) des Knochens relativ zueinander ausgerichtet werden sollen.

2. Verfahren nach dem vorhergehenden Anspruch, wobei als erstes (3) und zweites (4) Fragment des Knochens durch Fraktur oder Bruch oder Auseinanderbrechen des Knochens entstandene Fragmente (3, 4) eines Femurs, einer Tibia, eines Humerus oder einer Fibula verwendet werden.

3. Verfahren nach einem der vorhergehenden Ansprüche, wobei als charakteristische Landmarke (6a, 6b) des ersten (3) und zweiten Fragments (4) des Knochens die linea aspera des Femur verwendet wird.

4. Verfahren nach einem der vorhergehenden Ansprüche, wobei die Ultraschallvorrichtung (1) mittels des Navigationssystems (2) an eine vorgegebene oder ermittelbare Position navigiert wird, um die mindestens eine Landmarke (6a, 6b) des ersten Fragments (3) und des zweiten Fragments (4) zu erfassen.

5. Vorrichtung zum Ermitteln von Ausrichtungsinformationen, welche angeben, wie ein erstes Fragment (3) und ein zweites Fragment (4) eines Knochens relativ zueinander ausgerichtet werden sollen, wobei das erste (3) und das zweite Fragment (4) des Knochens an einer Verbindungsstelle (5) miteinander verbunden werden können, umfassend:
a) eine Ultraschallvorrichtung (1), mittels welcher
- eine charakteristische Landmarke (6a) auf dem Umfang oder der Außenseite des ersten Fragments (3) des Knochens in einem Bereich um die Verbindungsstelle (5) zwischen dem ersten (3) und dem zweiten Fragment (4) des Knochens erfasst werden kann und
- mindestens eine korrespondierende charakteristische Landmarke (6b) auf dem Umfang oder der Außenseite des zweiten Fragments (4) des Knochens in dem Bereich um die Verbindungsstelle (5) zwischen dem ersten (3) und dem zweiten Fragment (4) des Knochens, das heißt in einem Abstand von weniger als einem Zentimeter oder von ein, zwei, drei, vier, oder fünf Zentimeter zu einer Fraktur, erfasst werden kann, und
b) ein Navigationssystem (2) mit einer mit dem Navigationssystem (2) verbundenen oder in das Navigationssystem (2) integrierten Recheneinheit (2a), wobei mittels des Navigationssystems (2)
- die Position und/oder Orientierung der Ultraschallvortichtung (1) im Raum mit Hilfe einer an der Ultraschallvorrichtung (1) angeordneten Trackingreferenz (1a) ermittelt werden kann,
- die Position und/oder Orientierung des ersten (3) und des zweiten Fragments (4) des Knochens im Raum mit Hilfe von an dem ersten Fragment (3) und an dem zweiten Fragment (4) des Knochens angeordneten Trackingreferenzen (3a, 4a) ermittelt werden kann und
- die Position und/oder Orientierung der Landmarken (6a, 6b) im Raum basierend auf der Position und Orientierung der Ultraschallvorrichtung (1) und der Position und/oder Orientierung des ersten (3) und zweiten Fragments (4) des Knochens ermittelt werden kann.
so dass, basierend auf dem durch einen Benutzer in die Rechnereinheit (2a) eingegebene Lageverhältnis der Landmarken (6a, 6b) und der Position und/oder Orientierung der mindestens einen Landmarke (6a) auf dem ersten Fragment (3) des Knochens und der Position und/oder Orientierung der mindestens einen Landmarke (6b) auf dem zweiten Fragment (4) des Knochens, die Ausrichtungsinformationen ermittelt werden, welche angeben wie das erste (3) und das zweite Fragment (4) des Knochens relativ zueinander ausgerichtet werden sollen.

6. Vorrichtung nach dem vorhergehenden Anspruch, weiter umfassend eine mit der Ultraschallvorrichtung (1) verbundene Anzeigevorrichtung (8) auf welcher Ultraschallaufnahmen und/oder die ermittelten Ausrichtungsinformationen angezeigt werden können.

7. Vorrichtung nach einem der zwei vorhergehenden Ansprüche, wobei mittels des Navigationssystems (2) die Ultraschallvorrichtung (1) an vorgegebene oder ermittelte Positionen zur Aufnahme der mindestens einen Landmarke (6a) des ersten Fragments (3) und der mindestens einen Landmarke (4) des zweiten Fragments (4) des Knochens navigiert werden kann.

8. Computerprogramm, welches, wenn es in dem Navigationssystem von Ansprüch 5 geladen ist oder auf ihm läuft, das Verfahren nach einem der Ansprüche 1-4 durchführt.

9. Programmspeichermedium oder Computerprogrammprodukt mit dem Programm nach dem vorhergehenden Anspruch.

## Claims

1. A method for ascertaining alignment information which indicates how a first fragment (3) and a second fragment (4) of a bone are to be aligned relative to each other, wherein the first (3) and second (4) fragments of the bone can be connected to each other at a connecting point (5),
a) wherein the spatial position and/or orientation of the first (3) and second (4) fragments of the bone are ascertained by means of a navigation system (2) with the aid of tracking references (3a, 4a) arranged on the first fragment (3) and the second fragment (4) of the bone,
b) wherein by means of an ultrasound device (1), the spatial position and/or orientation of which is ascertained by means of the navigation system (2) with the aid of a tracking reference (1a) arranged on the ultrasound device (1), at least one characteristic landmark (6a) on the circumference or outer side of the first fragment (3) of the bone is detected in an area around the connecting point (5) between the first (3) and second (4) fragments of the bone, i.e. at a distance of less than one centimetre or a distance of one, two, three, four or five centimetres from a fracture, and
c) wherein at least one corresponding characteristic landmark (6b) on the circumference or outer side of the second fragment (4) of the bone is detected in the area around the connecting point between the first (3) and second (4) fragments of the bone by means of the ultrasound device (1);
d) wherein the spatial position and/or orientation of the landmarks (6a, 6b) is ascertained, based on the position and/or orientation of the ultrasound device (1) and the position and/or orientation of the first (3) and second (4) fragments of the bone, by a computational unit (2a) of the navigation system (2); and
e) wherein a positional relationship of the landmarks (6a, 6b) relative to each other is inputted into the computational unit (2a) of the navigation system (2) by a user,
f) such that the alignment information which indicates how the first (3) and second (4) fragments of the bone are to be aligned relative to each other is ascertained, based on the predetermined positional relationship of the landmarks (6a, 6b) and the position and/or orientation of the at least one landmark (6a) on the first fragment (3) of the bone and the position and/or orientation of the at least one landmark (6b) on the second fragment (4) of the bone.

2. The method according to the preceding claim, wherein fragments (3, 4) of a femur, tibia, humerus or fibula, created by a fracture or breaking apart of the bone, are used as the first (3) and second (4) fragments of the bone.

3. The method according to any one of the preceding claims, wherein the linea aspera of the femur is used as a characteristic landmark (6a, 6b) of the first (3) and second (4) fragments of the bone.

4. The method according to any one of the preceding claims, wherein the ultrasound device (1) is navigated to a predetermined or ascertainable position by means of the navigation system (2), in order to detect the at least one landmark (6a, 6b) of the first (3) and second (4) fragments.

5. A device for ascertaining alignment information which indicates how a first fragment (3) and a second fragment (4) of a bone are to be aligned relative to each other, wherein the first (3) and second (4) fragments of the bone can be connected to each other at a connecting point (5), said device comprising:
a) an ultrasound device (1), by means of which
- a characteristic landmark (6a) on the circumference or outer side of the first fragment (3) of the bone can be detected in an area around the connecting point (5) between the first (3) and second (4) fragments of the bone, and
- at least one corresponding characteristic landmark (6b) on the circumference or outer side of the second fragment (4) of the bone can be detected in the area around the connecting point (5) between the first (3) and second (4) fragments of the bone, i.e. at a distance of less than one centimetre or a distance of one, two, three, four or five centimetres from a fracture; and
b) a navigation system (2) comprising a computational unit (2a) which is connected to the navigation system (2) or integrated into the navigation system (2), wherein by means of the navigation system (2),
- the spatial position and/or orientation of the ultrasound device (1) can be ascertained with the aid of a tracking reference (1a) arranged on the ultrasound device (1),
- the spatial position and/or orientation of the first (3) and second (4) fragments of the bone can be ascertained with the aid of tracking references (3a, 4a) arranged on the first fragment (3) and the second fragment (4) of the bone, and
- the spatial position and/or orientation of the landmarks (6a, 6b) can be ascertained, based on the position and/or orientation of the ultrasound device (1) and the position and/or orientation of the first (3) and second (4) fragments of the bone,
such that the alignment information which indicates how the first (3) and second (4) fragments of the bone are to be aligned relative to each other is ascertained, based on the positional relationship of the landmarks (6a, 6b) inputted into the computational unit (2a) by a user and the position and/or orientation of the at least one landmark (6a) on the first fragment (3) of the bone and the position and/or orientation of the at least one landmark (6b) on the second fragment (4) of the bone.

6. The device according to the preceding claim, further comprising a display device (8) which is connected to the ultrasound device (1) and on which ultrasound recordings and/or the ascertained alignment information can be displayed.

7. The device according to any one of the preceding two claims, wherein by means of the navigation system (2), the ultrasound device (1) can be navigated to predetermined or ascertained positions in order to record the at least one landmark (6a) of the first fragment (3) and the at least one landmark (6b) of the second fragment (4) of the bone.

8. A computer program which, when it is loaded on the navigation system of claim 5 or is running on the navigation system of claim 5, performs the method according to any one of claims 1 to 4.

9. A program storage medium or computer program product comprising the program according to the preceding claim.

## Revendications

1. Procédé pour déterminer des informations d'alignement indiquant la manière dont un premier fragment (3) et un second fragment (4) d'un os doivent être alignés l'un par rapport à l'autre, le premier fragment (3) et le second fragment (4) de l'os pouvant être raccordés l'un à l'autre à un emplacement de jonction (5),
a) dans lequel la position et/ou l'orientation spatiale des premier (3) et second (4) fragments de l'os est déterminée à l'aide de repères de suivi (3a, 4a) disposés sur le premier fragment (3) et sur le second fragment (4) de l'os au moyen d'un système de navigation (2),
b) dans lequel, en utilisant un dispositif à ultrasons (1) dont la position et/ou l'orientation spatiale est déterminée à l'aide d'un repère de suivi (1a) disposé sur le dispositif à ultrasons (1) au moyen du système de navigation (2), au moins un point de repère caractéristique (6a) est détecté sur la circonférence ou le côté extérieur du premier fragment (3) de l'os dans la zone autour de l'emplacement de jonction (5) entre les premier (3) et second (4) fragments de l'os, c'est-à-dire à une distance inférieure à un centimètre ou à un, deux, trois, quatre ou cinq centimètres d'une fracture, et
c) dans lequel, en utilisant le dispositif à ultrasons (1), au moins un point de repère caractéristique correspondant (6b) est détecté sur la circonférence ou le côté extérieur du second fragment (4) de l'os dans la zone autour de l'emplacement de jonction entre les premier (3) et second (4) fragments de l'os,
d) dans lequel la position et/ou l'orientation spatiale des points de repère (6a, 6b) est déterminée sur la base de la position et/ou de l'orientation du dispositif à ultrasons (1) et de la position et/ou de l'orientation des premier (3) et second (4) fragments de l'os par une unité de calcul (2a) du système de navigation (2), et
e) dans lequel une relation de position des points de repère (6a, 6b) l'un par rapport à l'autre est entrée par un utilisateur dans l'unité de calcul (2a) du système de navigation (2),
f) de telle sorte que, sur la base de la relation de position prédéfinie des points de repère (6a, 6b) et de la position et/ou de l'orientation du au moins un point de repère (6a) sur le premier fragment (3) de l'os et/ou de la position et/ou de l'orientation du au moins un point de repère (6b) sur le second fragment (4) de l'os, les informations d'alignement sont déterminées, lesquelles indiquent la manière dont les premier (3) et second (4) fragments de l'os doivent être alignés l'un par rapport à l'autre.

2. Procédé selon la revendication précédente, dans lequel des fragments (3, 4) d'un fémur, d'un tibia, d'un humérus ou d'un péroné résultant d'une fracture ou d'une rupture sont utilisés comme premier (3) et second (4) fragments de l'os.

3. Procédé selon l'une quelconque des revendications précédentes, dans lequel la ligne âpre du fémur est utilisée comme point de repère caractéristique (6a, 6b) des premier (3) et second (4) fragments de l'os.

4. Procédé selon l'une quelconque des revendications précédentes, dans lequel le dispositif à ultrasons (1) est déplacé au moyen du système de navigation (2) jusqu'à une position prédéfinie ou pouvant être déterminée, afin de détecter le au moins un point de repère (6a, 6b) des premier (3) et second (4) fragments.

5. Dispositif pour déterminer des informations d'alignement qui indiquent la manière dont un premier fragment (3) et un second fragment (4) d'un os doivent être alignés l'un par rapport à l'autre, dans lequel les premier (3) et second (4) fragments de l'os peuvent être raccordés l'un à l'autre à un premier emplacement de jonction (5), comportant :
a) un dispositif à ultrasons (1) au moyen duquel
- un point de repère caractéristique (6a) peut être détecté sur la circonférence ou le côté extérieur du premier fragment (3) de l'os dans la zone autour de l'emplacement de jonction (5) entre les premier (3) et second (4) fragments de l'os et
- au moins un point de repère caractéristique correspondant (6b) peut être détecté sur la circonférence ou le côté extérieur du second fragment (4) de l'os dans la zone autour de l'emplacement de jonction (5) entre les premier (3) et second (4) fragments de l'os, c'est-à-dire à une distance inférieure à un centimètre ou à un, deux, trois, quatre ou cinq centimètres d'une fracture, et
b) un système de navigation (2) comportant une unité de calcul (2a) reliée au système de navigation (2) ou intégrée dans le système de navigation (2), dans lequel au moyen du système de navigation (2)
- la position et/ou l'orientation spatiale du dispositif à ultrasons (1) peut être déterminée à l'aide d'un repère de suivi (1a) disposé sur le dispositif à ultrasons (1),
- la position et/ou l'orientation spatiale des premier (3) et second (4) fragments de l'os peut être déterminée à l'aide de repère de suivi (3a, 4a) disposé sur le premier fragment (3) et sur le second fragment (4) de l'os et
- la position et/ou l'orientation spatiale des points de repère (6a, 6b) peut être déterminée sur la base de la position et de l'orientation du dispositif à ultrasons (1) et de la position et/ou de l'orientation des premier (3) et second (4) fragments de l'os,
de telle sorte que, sur la base de la relation de position des points de repère (6a, 6b) entrée par un utilisateur dans l'unité de calcul (2a) et de la position et/ou de l'orientation du au moins un point de repère (6a) sur le premier fragment (3) de l'os et de la position et/ou de l'orientation du au moins un point de repère (6b) sur le second fragment (4) de l'os, les informations d'alignement sont déterminées, lesquelles indiquent la manière dont les premier (3) et second (4) fragments de l'os doivent être alignés l'un par rapport à l'autre.

6. Dispositif selon la revendication précédente, comportant en outre un dispositif d'affichage (8) relié au dispositif à ultrasons (1), sur lequel peuvent être affichées des enregistrements par ultrasons et/ou les informations d'alignement déterminées.

7. Dispositif selon l'une quelconque des deux revendications précédentes, dans lequel au moyen du système de navigation (2), le dispositif à ultrasons (1) peut être déplacé jusqu'à des positions prédéfinies ou déterminées afin d'enregistrer le au moins un point de repère (6a) du premier fragment (3) et le au moins un point de repère (6b) du second fragment (4) de l'os.

8. Programme informatique qui, lorsqu'il est chargé dans le système de navigation de la revendication 5 ou lorsqu'il s'exécute sur celui-ci, met en oeuvre le procédé selon l'une quelconque des revendications 1 à 4.

9. Support d'enregistrement de programme ou produit de programme informatique comportant le programme selon la revendication précédente.
